# DEMANDE DE BREVET EUROPEEN

(11) **EP 2 628 788 A1**
(43) Date de publication de la demande: **21.08.2013**
(21) Numéro de dépôt: 13155830.6
(22) Date de dépôt: 19.02.2013
(51) Int. Cl.: C12M 1/107

(54) **Procédé et installation de préparation d'un substrat de méthanisation**

(30) Priorité: 20.02.2012 FR 1251536
(71) Demandeur: Methaneo, 75014 Paris (FR)
(72) Inventeur: Mercier, Yann, 92800 PUTEAUX (FR); Guitonneau, Fabien, 92120 MONTROUGE (FR)
(74) Mandataire: Blot, Philippe Robert Emile

(57) **Abrégé**

Le procédé comprend :
- la dilution de la biomasse fibreuse dans un liquide pour former un mélange (M) comprenant les produits fibreux en suspension dans le liquide ;
- l'hydrolyse des produits fibreux dans le mélange ;
- la séparation de la matière sèche (MS) en suspension humidifiée du liquide (L) ; et
- la formation d'un substrat de méthanisation (S) adapté pour la méthanisation à partir de la matière sèche(MS) séparée du liquide (L).

## Description

L'invention concerne un procédé et une installation de préparation d'un substrat de méthanisation à partir de biomasse fibreuse, le substrat de méthanisation étant destiné à une valorisation énergétique par méthanisation. La méthanisation de biomasse fibreuse permet notamment la production de biogaz.

La méthanisation ou digestion anaérobie est un processus biologique naturel de dégradation de la matière organique par des bactéries. L'un des produits de cette dégradation est le biogaz, principalement composé de méthane qui est un gaz énergétique.

Ce processus naturel a trouvé des applications industrielles, représentant un moyen compétitif de production d'énergie à partir de matière organique, et principalement de déchets ou de co-produits d'activités humaines industrielles ou agricoles.

La plupart de la matière organique produite par ces activités provient de l'élevage et est composée pour sa majeure partie de fumier. Le fumier correspond à une pratique d'élevage reposant sur l'utilisation de paille pour la constitution de litière, participant au confort et donc à la productivité de l'élevage.

Une partie conséquente du potentiel de production de biogaz à partir de co-produits d'activités industrielles et agricoles, indépendamment du potentiel de valorisation de cultures spécifiques dédiées à la méthanisation, provient donc du fumier pailleux produit par l'élevage, et de la paille, produite notamment à cette même fin de valorisation énergétique.

Ces produits, riches en matière organique biodégradable, présentent les caractéristiques suivantes:
- une forte concentration de paille fibreuse de longue taille, assouplie par la matière fécale à laquelle elle est mélangée ; et
- une hétérogénéité des caractéristiques des différents produits pouvant être intégrés dans la filière de valorisation, notamment de la siccité
- une présence importante et aléatoire d'éléments indésirables liée :
   - à la récolte de la paille sur des surfaces cultivées rocailleuses (présence de pierre et de sable) ;
   - à la difficulté du contrôle du fumier, produit dans des fermes aux pratiques traditionnelles (présence de pièces de machines agricoles, sceaux, pièces métalliques, parpaings, etc.) ;

Le fumier est un produit difficilement méthanogène, si la paille qui le compose n'est pas convenablement broyée, permettant une surface spécifique de dégradation optimisée.

L'absence de constance dans la siccité du produit valorisé par méthanisation rend difficile un dosage précis de la matière organique, indispensable au bon équilibre de la flore bactérienne.

La présence d'éléments indésirables dangereux pour les équipements installés induit des aléas d'exploitation incompatibles avec les impératifs de production d'une installation industrielle.

Les éléments indésirables, présents dans tous les types de biomasse solides aujourd'hui valorisés dans des unités de méthanisation (ensilage, fumier, etc.) sont souvent détectés de manière visuelle, ceci induisant une rupture de charge dans le procédé d'alimentation de l'unité de méthanisation, imposant une intervention humaine.

Il se trouve que les biomasses fibreuses, telles que le fumier et la paille, représentent aujourd'hui rarement l'essentiel d'un gisement valorisé dans une unité de méthanisation, qui quand ils le sont, sont broyés en voie liquide, après mélange avec la biomasse dominante.

Une vraie problématique industrielle se crée aujourd'hui autour d'une possibilité de meilleure valorisation de ce type de produit.

Un des buts de l'invention est de permettre une meilleure valorisation de la biomasse fibreuse par méthanisation, notamment de la paille et du fumier pailleux.

A cet effet, l'invention propose un procédé de préparation d'un substrat de méthanisation à partir de biomasse fibreuse solide, le procédé comprenant :
- la dilution de la biomasse fibreuse dans un liquide pour former un mélange comprenant les produits fibreux en suspension dans le liquide ;
- l'hydrolyse des produits fibreux dans le mélange ;
- la séparation de la matière sèche en suspension humidifiée du liquide; et
- la formation d'un substrat de méthanisation adapté pour la méthanisation à partir de la matière sèche séparée du liquide.

Selon d'autres modes de mise en oeuvre, le procédé comprend une ou plusieurs des caractéristiques suivantes, prise(s) isolément ou selon toute(s) combinaison(s) techniquement possible(s) :
- la biomasse est diluée de façon que le mélange contienne de 0.5% à 15% en masse de matière sèche, de préférence de 5% à 10% en masse de matière sèche ;
- une étape de broyage de la biomasse fibreuse avant dilution ;
- le stockage de la biomasse fibreuse broyée et la dilution progressive de la biomasse broyée ;
- le broyage de la matière sèche du mélange en phase liquide avant la séparation ;
- le broyage de la matière sèche après séparation d'avec le liquide ;
- la séparation de la matière sèche et du liquide est effectuée par dégrillage, pressage et/ou centrifugation ;
- la matière sèche séjourne en moyenne 1 à 10 jours dans le liquide ;
- du liquide séparé de la matière sèche et/ou du mélange est (sont) utilisé(s) pour diluer la matière sèche de manière à contrôler la siccité du substrat de méthanisation.
- le procédé comporte l'ajout de produits auxiliaires comme source de biomasse, par exemple de la biomasse d'origine végétale animale ou fongique, ne nécessitant pas de broyage avant dilution.

L'invention concerne également une installation de préparation d'un substrat de méthanisation à partir de biomasse fibreuse des produits fibreux, comprenant :
- une fosse de dilution de la biomasse fibreuse pour former un mélange contenant de la matière sèche fibreuse en suspension dans un liquide et d'hydrolyse de la matière sèche dans le mélange ; et
- un dispositif de séparation de phase pour séparer de la matière sèche du liquide à partir du mélange.

Selon d'autres modes de réalisation, l'installation comprend un ou plusieurs des caractéristiques suivantes, prise(s) isolément ou selon toute(s) combinaison(s) techniquement possible(s) :
- un dispositif de broyage amont pour broyer la biomasse fibreuse avant sa dilution.
- un dispositif de broyage aval pour broyer la matière sèche extraite du mélange ;
- un dispositif de broyage en phase liquide pour broyer la matière sèche dans le mélange ;
- un dispositif d'ajustement de siccité pour mélanger la matière sèche à du liquide séparé de la matière sèche par le dispositif de séparation et/ou du mélange contenu dans la fosse.

L'invention concerne encore un système de production de biogaz comprenant une installation de préparation telle que définie ci-dessus et un digesteur anaérobie de méthanisation alimenté en substrat de méthanisation par l'installation de préparation.

L'invention et ses avantages seront mieux compris à la lecture de la description qui va suivre, donnée uniquement à titre d'exemple et faite en référence aux dessins annexés, sur lesquels :
- la Figure 1 est une vue schématique d'une installation de production de biogaz ;
   et
- les Figures 2 à 5 sont des vues schématiques de dispositifs de séparation de phase pour récupérer de la matière sèche dans un liquide.

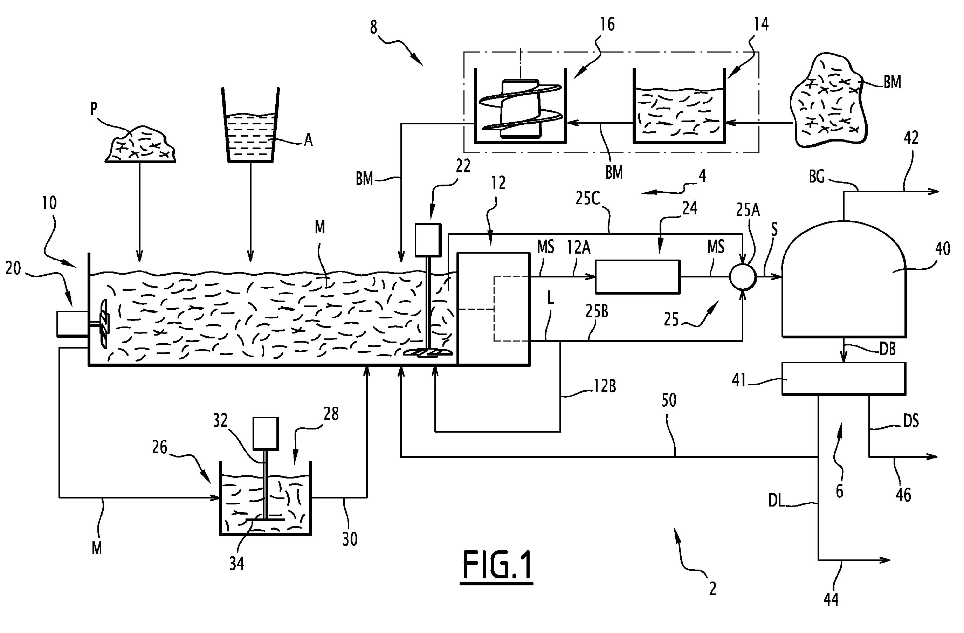

La Figure 1 illustre un exemple d'une installation de production de biogaz 2 pour la production de biogaz BG à partir de biomasse fibreuse BM solide.

Le terme « biogaz » désigne de manière générale un gaz contenant du méthane.

Le terme « biomasse » désigne de manière générale des matières organiques végétales, animales ou fongiques permettant la production d'énergie, notamment en permettant la production de biogaz par méthanisation.

Le terme « biomasse fibreuse » désigne de la biomasse contenant de la matière organique fibreuse, généralement sous la forme de fibres lignocellulosiques. Les fibres végétales sont en effet composées de cellulose, d'hémicellulose et de lignine. La biomasse fibreuse peut aussi être désignée par le terme « biomasse ligneuse » ou « biomasse lignocellulosique ».

L'installation de production de biogaz 2 comprend une installation de préparation 4 pour la préparation d'un substrat de méthanisation S solide à partir de biomasse fibreuse BM solide et une installation de méthanisation ou digesteur 6 pour la production de biogaz BG par digestion anaérobie du substrat de méthanisation S fourni par l'installation de préparation 4.

L'installation de préparation 4 comprend un dispositif de prétraitement 8 pour la réception de la biomasse fibreuse BM, une fosse 10 pour la dilution, l'hydrolyse et l'homogénéisation de la biomasse fibreuse BM, et un dispositif de séparation de phase 12 pour séparer la matière organique solide hydrolysée du liquide.

Le dispositif de prétraitement 8 est adapté pour recevoir de la biomasse fibreuse BM en phase solide, la broyer et la stocker.

La biomasse fibreuse BM est par exemple de la paille et/ou du fumier pailleux. Le fumier pailleux est un mélange de fumier et de paille récupéré sur les exploitations agricoles d'élevages de touts types (ovin, bovin, porcin...).

La paille et le fumier pailleux sont susceptibles de contenir des éléments indésirables. En pratique, il est courant de retrouver du sable et des cailloux dans les fumiers pailleux en provenance d'exploitations agricoles installées sur des terrains rocailleux. Il est aussi possible de retrouver des éléments indésirables de tous types et de toutes dimensions (pièces métalliques d'outils agricoles, pièces en matière plastique, parpaings, briques...) potentiellement préjudiciables pour l'installation de préparation du substrat de méthanisation.

Le dispositif de prétraitement 8 comprend un dispositif de stockage 14 pour stocker la biomasse fibreuse BM ainsi qu'un dispositif de broyage amont 16 pour broyer et mélanger la biomasse fibreuse BM en phase solide.

La cuve de stockage 14 permet le stockage de la biomasse fibreuse.

Le dispositif de broyage amont 16 est de tout type approprié pour le broyage d'une biomasse solide contenant des produits indésirables. Le dispositif de broyage amont 16 est alimenté en biomasse solide par le dispositif de stockage 14.

Dans un mode de réalisation, le dispositif de broyage amont 16 comprend au moins une trémie, par exemple une remorque trémie, munie d'une vis conique telles que celles utilisées dans les outils agricoles, par exemple dans les désileuses.

Le dispositif de stockage 14 et le dispositif de broyage amont 16 sont représentés séparés. En variante, ils sont réunis dans une unité fonctionnelle de prétraitement.

La fosse 10 définit un volume interne de réception de liquide et de la biomasse fibreuse BM broyée de façon à former un mélange M contenant de la matière sèche MS constituée de matière organique provenant de la biomasse fibreuse BM broyée, en suspension dans un liquide L. La matière sèche MS issue de la biomasse fibreuse BM se présente essentiellement sous la forme de fibres lignocellulosiques.

La fosse 10 permet également la réception de produits supplémentaires P comme source auxiliaire de biomasse ou d'additifs A notamment liquides.

La fosse 10 permet une séparation gravitaire des éléments indésirables présents dans la fosse 10. Les éléments indésirables sont généralement plus denses que l'eau et se déposent par gravité sur le fond de la fosse 10.

La fosse 10 comprend au moins un agitateur pour agiter le mélange M. La fosse 10 de dilution peut comprend un agitateur horizontal pour générer une circulation suivant une direction horizontale dans la cuve, un agitateur vertical pour générer une circulation fluidique suivant une direction verticale dans la cuve et/ou un agitateur incliné pour générer une circulation fluidique suivant une direction inclinée par rapport à l'horizontal et à la verticale. Dans l'exemple illustré, la cuve comprend un agitateur horizontal 20 et un agitateur vertical 22.

Chaque agitateur comprend par exemple une hélice d'agitateur plongeant dans le mélange M et entraînée en rotation par un moteur d'agitateur. L'axe de rotation de l'hélice d'agitateur correspondant à la direction d'agitation de l'agitateur.

Le ou chaque agitateur permet d'assurer des mouvements fluidique de telle manière que la matière sèche MS se répartisse dans le liquide L et soit mouillée par le liquide L. Les remous provoqués par les agitateurs sont suffisants pour remuer la matière organique sans soulever les éléments indésirables du fond de la fosse 10.

La dilution de la biomasse fibreuse BM en phase liquide permet d'hydrolyser les fibres provenant de la biomasse fibreuse BM. L'hydrolyse des fibres conduit à un défibrage au moins partiel des fibres. Ce défibrage facilite les étapes de préparation ultérieures du substrat de méthanisation S, en amont du digesteur 6, et/ou l'action bactérienne dans le digesteur 6.

L'hydrolyse est favorisée par un séjour prolongé des fibres dans l'eau. La fosse 10 de dilution est dimensionnée pour la dilution d'une quantité journalière de biomasse fibreuse supérieure à la quantité nécessaire au fonctionnement du digesteur. Il en résulte que la matière sèche MS provenant de la biomasse fibreuse diluée dans la fosse 10 de dilution séjourne pendant une longue période de temps dans le liquide. Ceci permet l'hydrolyse avancée des fibres. La fosse 10 est dimensionnée de préférence pour une durée moyenne de séjour de la matière organique de 1 à 10 jours.

L'hydrolyse est favorisée par un pH acide (pH < 7). Ainsi, afin de favoriser l'hydrolyse, il est possible de contrôler le pH du mélange, par exemple par ajout d'acide dans le mélange pour en diminuer le pH. De préférence, le pH est maintenu entre 4 et 8.

L'hydrolyse est favorisée par la chaleur. Ainsi, afin de favoriser l'hydrolyse, il est possible de chauffer le mélange M dans la fosse 10 pour le maintenir à une certaine température, de préférence une température comprise entre 30 et 60°C.

Le dispositif de séparation de phase 12 permet de séparer la phase solide (matière sèche MS) de la phase liquide (liquide L) du mélange M. Le dispositif de séparation de phase 12 prélève du mélange M dans la fosse 10 et sépare la matière sèche MS du liquide L.

Le dispositif de séparation de phase 12 comprend une sortie de matière sèche 12A fournissant la matière sèche MS. Le substrat S est obtenu principalement à partir de la matière sèche.

Le dispositif de séparation de phase 12 comprend une sortie de liquide 12B aménagée de façon à ce que le liquide L séparé de la matière sèche MS soit réintroduit dans la fosse 10.

L'installation de préparation 4 comprend en option un dispositif de broyage aval 24, pour broyer la matière sèche MS hydrolysée extraite de la fosse 10 par le dispositif de séparation de phase 12. Le dispositif de broyage aval 24 est disposé sur la sortie de matière sèche 12A du dispositif de séparation de phase 12.

Le broyage aval, après extraction des éléments indésirables et hydrolyse de la matière sèche, est de préférence un broyage fin permettant d'obtenir une granulométrie moyenne de fibres d'environ 15 mm dans la longueur de la fibre et 2 mm dans la largeur de la fibre.

L'installation de préparation 4 comprend un dispositif d'ajustement de siccité 25 pour ajuster la siccité du substrat de méthanisation S formé à partir de la matière sèche MS. Le dispositif d'ajustement de siccité 25 comprend un mélangeur 25A pour mélanger la matière sèche MS issue du dispositif de séparation 12, et éventuellement broyée par le dispositif de broyage aval 24, avec du liquide L issu du dispositif de séparation 12 et/ou du mélange M pris directement dans la fosse 10, et former le substrat de méthanisation S.

Le mélangeur 25A est alimenté en matière sèche MS par la sortie de matière sèche 12A. Le mélangeur 25A est disposé sur la sortie de matière sèche 12A en aval dispositif de broyage aval 24. Le mélangeur 25A est en outre alimenté en liquide L par une ligne d'alimentation en liquide 25B raccordée à la sortie de liquide dispositif de séparation de phase 12 et/ou en mélange M par une ligne d'alimentation en mélange 25C s'alimentant dans la fosse 10.

Le substrat de méthanisation S est ainsi formé principalement de matière sèche MS, éventuellement broyée, à laquelle est éventuellement ajouté du liquide L et/ou du mélange M.

En option, l'installation de préparation 4 comprend un dispositif de broyage en phase liquide 26 pour broyer la matière sèche MS du mélange M.

Tel qu'illustré sur la Figure 1, le dispositif de broyage en phase liquide 26 comprend un broyeur en phase liquide 28 disposé sur une conduite de recirculation 30 ayant une extrémité s'alimentant dans la fosse 10 et l'autre extrémité débouchant dans la fosse 10.

Le dispositif de broyage en phase liquide 26 comprend par exemple un plongeur de broyage 32 comprenant au moins une lame 34 de broyage rotative plongée dans le mélange pour broyer les fibres contenues dans le mélange du fait de la rotation à vitesse élevée de la lame.

Le dispositif de broyage en phase liquide 26 est actionné de préférence après un laps de temps après l'introduction de biomasse dans la fosse 10 pour assurer le dépôt gravitaire des éléments indésirables dans le fond de la cuve, sans que ces éléments indésirables ne soient entraînés vers le dispositif de broyage en phase liquide.

Le substrat de méthanisation S préparé par l'installation de préparation 4 alimente le digesteur 6. Dans le cas présent, le digesteur 6 est alimenté en substrat de méthanisation S par la sortie du mélangeur 25A.

Le digesteur 6 comprend une enceinte de méthanisation 40 dans laquelle le substrat de méthanisation S est introduit pour être soumis à une digestion anaérobie par des bactéries et un séparateur de phase de digestat 41.

Le digesteur 6 fournit en sortie du biogaz BG contenant du méthane par une ligne de sortie 42, du digestat liquide DL par une ligne de digestat liquide 44, et du digestat solide DS par une ligne de digestat solide 46. Plus spécifiquement, l'enceinte de méthanisation 40 fournit en sortie le biogaz BG et un digestat brut DB, et le séparateur de phase de digestat 41 sépare le digestat brut DB de manière à fournir, d'une part, le digestat liquide DL et, d'autre part, le digestat solide DS.

Le biogaz BG est valorisé, par exemple pour la production d'énergie mécanique ou électrique. Le digestat liquide DL, ou au moins une fraction du digestat liquide, est recirculé(e) dans la fosse 10 par l'intermédiaire d'une ligne de recirculation 50. Le digestat solide DS est extrait et valorisé.

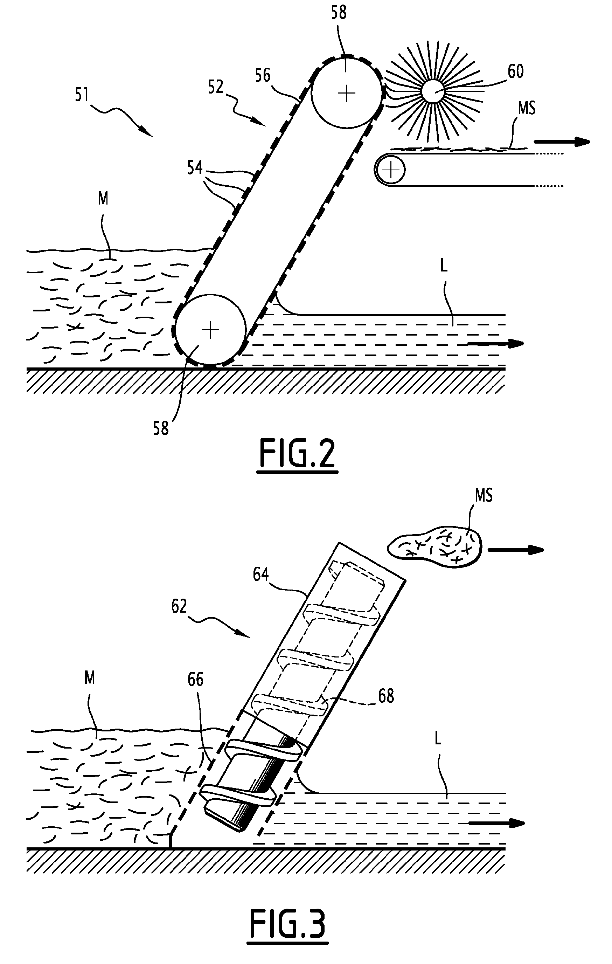

Le dispositif de séparation de phase 12 est de tout type approprié pour séparer de la biomasse fibreuse d'un liquide. Des dispositifs de séparation de phase appropriés sont illustrés sur les Figure 2 à 5.

Tel qu'illustré sur la Figure 2, le dispositif de séparation de phase 12 se présente sous la forme d'un dégrilleur 51 comprenant un écran 52 de filtrage perforé en forme de bande sans fin. L'écran 52 est formé d'une pluralité de plaques 54 de filtrage perforées portées par une chaîne sans fin 56 et un dispositif d'entraînement de l'écran de filtrage comprenant des rouleaux 58 rotatif autour desquels la chaîne sans fin 56 est passée. Le dégrilleur 51 est disposé de telle sorte que l'écran 52 comprend une portion descendante descendant dans la fosse 10 et une portion montante sortant de la fosse 10.

Le dégrilleur 51 comprend en outre un organe de nettoyage, par exemple une brosse rotative 60, pour retirer la biomasse de la portion d'écran sortant de la fosse 10 et ainsi récupérer la matière sèche extraite de la fosse 10.

L'installation de préparation 4 comprend un dispositif d'aspiration 54 pour aspirer du mélange au travers de l'écran 52 du dégrilleur 51. Le dispositif d'aspiration 54 comprend une pompe (non représentée) pour aspirer le mélange au travers de l'écran 52. De préférence, la pompe refoule le liquide aspiré et filtré dans la fosse 10 de dilution par une conduite de retour.

En fonctionnement, le mélange est aspiré au travers de l'écran 52 du dégrilleur 51. Le mélange est filtré, la matière sèche MS restant accrochée à l'écran 52 et le liquide traversant l'écran 52. La matière sèche MS est emportée à l'extérieur de la fosse 10 du fait du déplacement de l'écran 52, et récupérée grâce à la brosse 60 qui retire la matière sèche MS de l'écran 52.

Tel qu'illustré sur la Figure 3, le dispositif de séparation de phase 12 se présente sous la forme d'un dispositif de filtrage à vis sans fin 62 comprenant un tube 64 incliné dont l'extrémité inférieure est munie d'un écran 66 fixe perforé, et une vis sans fin 68 rotative à l'intérieur du tube 64 de façon racler l'écran 66 et à remonter la matière sèche retenue par l'écran 66 hors de la fosse 10.

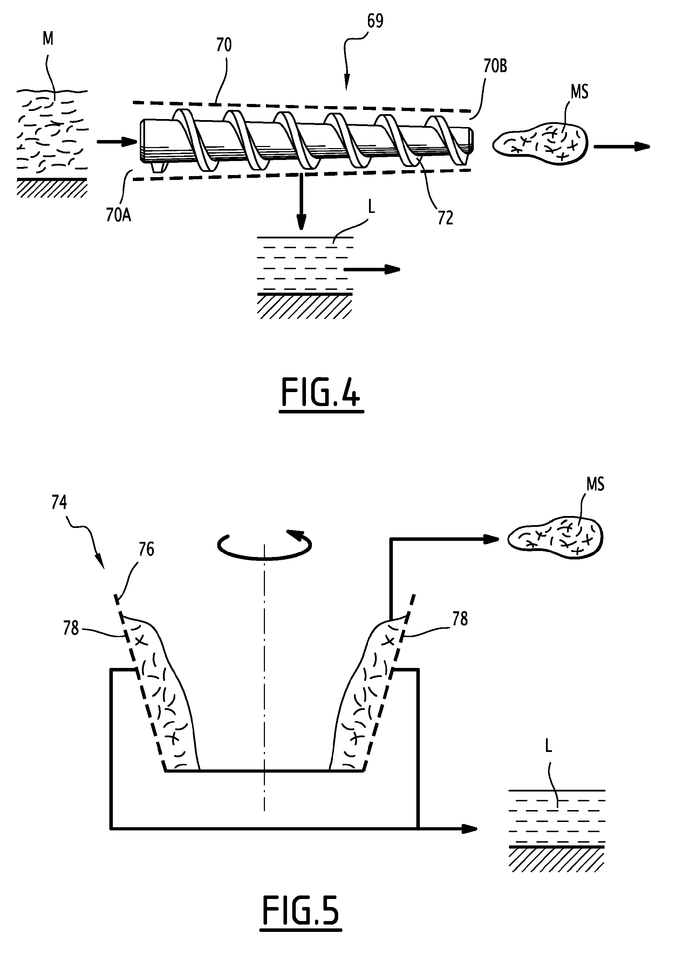

Tel qu'illustré sur la Figure 4, le dispositif de séparation de phase 12 se présente sous la forme d'une presse à vis sans fin 69 du type comprenant un tube 70 perforé présentant une entrée 70A et une sortie 70B et une vis sans fin 72 s'étendant à l'intérieur d'un tube 70 de façon à forcer le mélange introduit dans la vis sans fin vers la sortie, la fraction liquide du mélange étant évacuée par les perforation du tube 70 et la fraction sèche étant évacuée par la sortie 70B. La séparation de phase par pressage permet de récupérer une fraction solide relativement sèche.

Tel qu'illustré sur la Figure 5 le dispositif de séparation de phase 12 se présente sous la forme d'une centrifugeuse 74 comprenant une cuve de centrifugation 76 rotative à parois latérales 78 perforées, de telle manière que lors de la rotation de la cuve de centrifugation 76, la faction liquide traverse les parois latérales 78 tandis que la fraction solide est retenue par les parois latérale. Une centrifugeuse permet d'extraire une fraction liquide relativement claire.

Le procédé de préparation selon l'invention est mis en oeuvre lors du fonctionnement de l'installation de préparation 4.

Lors du fonctionnement, la biomasse fibreuse BM (en particulier paille et fumier pailleux) est introduite dans le dispositif de prétraitement 8. La biomasse fibreuse BM est stockée dans le dispositif de stockage 14. Elle subit ensuite, dans le dispositif de broyage amont 16, un broyage amont en phase solide. A ce stade du procédé de préparation, la biomasse fibreuse BM contient potentiellement des éléments indésirables. Le broyage amont est un broyage grossier permettant d'obtenir une granulométrie moyenne de fibres d'environ 50 mm dans la longueur de la fibre et 5 mm dans la largeur de la fibre.

La biomasse fibreuse BM broyée est introduite au fur et à mesure des besoins dans la fosse 10 de dilution.

La fosse 10 de dilution est par ailleurs alimentée en produits auxiliaires P formant des sources auxiliaires de biomasse et en additifs A liquides. Le liquide L est composé du liquide L recirculé par la sortie de recirculation 12B, d'autres composants additionnels tels que du digestat liquide DL provenant du digesteur 4 et recirculé par la ligne de recirculation 50, d'eau ou d'additifs A susceptibles d'améliorer la préparation de la biomasse afin de maximiser l'énergie extraite à partir de la biomasse fibreuse BM. Il est possible par exemple d'ajouter de l'urée ou de l'acide. Les produits auxiliaires P peuvent être de la biomasse d'origine végétale, animale ou fongique ne nécessitant par de broyage amont avant d'être introduit dans la fosse 10.

La biomasse fibreuse BM est diluée dans la fosse 10 de dilution et séjourne dans la fosse 10 de dilution de telle manière que la matière sèche MS, et en particulier les fibres qu'elle contient, soient hydrolysées par l'eau présente dans le liquide.

L'obtention d'une hydrolyse satisfaisante dépend notamment de la siccité du mélange M dans la fosse 10 et du temps de séjour moyen de la matière sèche MS dans la fosse 10.

La biomasse fibreuse BM est diluée de telle manière à maintenir une siccité contrôlée en fonction de la biomasse fibreuse BM utilisée, afin d'obtenir une hydrolyse satisfaisante des fibres contenues dans la biomasse. De préférence, la siccité dans la fosse 10 est maintenue entre 0.5% et 15%, encore de préférence maintenue entre 5% et 10%. La siccité est par exemple choisie environ égale à 5% pour de la biomasse fibreuse BM sous forme de fumier pailleux, et environ égale 10% pour de la biomasse fibreuse BM sous forme de paille.

La fosse 10 est dimensionnée en fonction du débit de matière organique nécessaire au digesteur de telle manière que le temps de séjour moyen de la matière organique dans la fosse 10 est compris entre 1 et 10 jours.

La dilution de la biomasse et son séjour prolongé dans la fosse 10 permettent le dépôt gravitaire des éléments indésirables dans le fond de la fosse 10. La biomasse fibreuse BM est ainsi purifiée des éléments indésirables susceptibles d'empêcher les autres étapes de préparation ultérieure tel que le broyage en phase liquide ou le broyage aval fin.

Le volume de mélange dans la fosse 10 assure une homogénéisation de la matière organique extraite de la fosse 10 par rapport aux lots de biomasse fibreuse BM introduits dans la fosse 10. La biomasse fibreuse introduite dans la fosse 10 peut provenir de différentes sources avec des compositions variées. La biomasse fibreuse BM peut provenir de différentes exploitations agricoles et/ou de différents élevages (ovin, bovin, porcin...) produisant des fumiers différents selon la pratique de chaque exploitant, le sol de chaque exploitation et/ou le type d'élevage. Etant donné le volume de la fosse 10 assurant un séjour moyen de plusieurs jours de la matière organique, en pratique, plusieurs arrivages de biomasse en provenance de plusieurs exploitations sont nécessaires au remplissage de la fosse 10, de sorte que les différents arrivages sont mélangés. La biomasse extraite de la fosse 10 est ainsi homogénéisée.

Le dispositif de broyage en phase liquide 26 optionnel permet de broyer les fibres présentes dans le mélange M, ce qui facilite encore leur hydrolyse. Le dispositif de broyage en phase liquide 24 permet un broyage plus fin que le broyage amont, notamment du fait qu'il s'effectue sur la matière sèche débarrassée des éléments indésirables.

Ensuite, le mélange M contenant la matière sèche MS hydrolysée et éventuellement broyée en phase liquide, est séparé en phase liquide et phase solide par le dispositif de séparation de phase 12. La séparation de phase permet de récupérer la matière sèche MS hydrolysée sous forme solide.

Le liquide L filtré est recirculé vers la fosse 10 ou mélangé avec la matière sèche MS, dans le mélangeur 25A, pour ajuster la siccité du substrat de méthanisation S. Du mélange M prélevé dans la fosse 10 peut également être mélangé à la matière sèche MS pour ajuster la siccité du substrat de méthanisation S.

Le dispositif de broyage aval 24 permet de réaliser un broyage fin sur la matière sèche MS hydrolysée, de façon à broyer les fibres plus finement.

Le substrat de méthanisation S ainsi obtenu contient des fibres finement broyées et hydrolysée, ce qui favorise l'action ultérieure des bactéries dans le digesteur 6 et assure une production de biogaz avec un rendement élevé.

La constance du substrat de méthanisation alimentant le digesteur influe sur le rendement du digesteur. Un substrat de méthanisation constant assure une meilleure stabilité de la population bactérienne et un meilleur rendement de production de biogaz. Il est notamment avantageux de maîtriser la siccité du substrat de méthanisation. L'installation de préparation de l'invention permet de maîtriser la siccité de la matière organique extraite de la fosse 10, et de mélanger si nécessaire cette matière organique avec du liquide provenant de la fosse 10 et/ou du mélange prélevé directement dans la fosse 10 avant d'alimenter le digesteur.

Ainsi, grâce à l'invention, il est possible de préparer un substrat de méthanisation à partir de biomasse fibreuse permettant une production efficace de biogaz dans un digesteur anaérobie

L'invention permet la soustraction de produits indésirables présents dans la biomasse fibreuse provenant d'exploitations agricoles, de créer un substrat de méthanisation broyé, défibré et constant en composition de matière sèche, permettant d'alimenter une filière de méthanisation avec un produit homogène, exempt de produits solides dangereux pour les équipements, et dont la biodégradabilité est maximisée, permettant des temps de séjour minimisée dans une filière de méthanisation.

Ce substrat peut être obtenu à partir de paille et de fumier pailleux comme source principales de biomasse, ce qui permet de valoriser efficacement à grande échelle ce type de biomasse largement disponible.

De plus l'invention permet de découpler l'alimentation de la filière de méthanisation en produit solide et en produit liquide et permet ainsi de s'adapter aux besoins d'alimentation et aux contraintes de l'installation.

## Revendications

1. Procédé de préparation d'un substrat de méthanisation à partir de biomasse fibreuse solide, le procédé comprenant :
- la dilution de la biomasse fibreuse dans un liquide pour former un mélange (M) comprenant les produits fibreux en suspension dans le liquide ;
- l'hydrolyse des produits fibreux dans le mélange ;
- la séparation de la matière sèche (MS) en suspension humidifiée du liquide (L) ;
et
- la formation d'un substrat de méthanisation (S) adapté pour la méthanisation à partir de la matière sèche(MS) séparée du liquide (L).

2. Procédé selon la revendication 1, dans lequel la biomasse est diluée de façon que le mélange contienne de 0.5% à 15% en masse de matière sèche, de préférence de 5% à 10% en masse de matière sèche.

3. Procédé selon l'une quelconque des revendications précédentes, comprenant une étape de broyage de la biomasse fibreuse avant dilution.

4. Procédé selon la revendication 3, comprenant le stockage de la biomasse fibreuse broyée et la dilution progressive de la biomasse broyée.

5. Procédé selon l'une quelconque des revendications précédentes, comprenant le broyage de la matière sèche du mélange en phase liquide avant la séparation.

6. Procédé selon l'une quelconque des revendications précédentes, comprenant le broyage de la matière sèche après séparation d'avec le liquide.

7. Procédé selon l'une quelconque des revendications précédentes, dans lequel la séparation de la matière sèche et du liquide est effectuée par dégrillage, pressage et/ou centrifugation.

8. Procédé selon l'une quelconque des revendications précédentes, comprenant l'ajout dans le liquide d'additifs (A) liquides, par exemple de l'urée ou de l'acide, et/ou de produits auxiliaires (P) comme source de biomasse, par exemple de la biomasse d'origine végétale, animale ou fongique, sans broyage avant dilution dans le liquide.

9. Procédé selon l'une quelconque des revendications précédentes, dans lequel du liquide séparé de la matière sèche et/ou du mélange est (sont) utilisé(s) pour diluer la matière sèche de manière à contrôler la siccité du substrat de méthanisation (S).

10. Installation de préparation d'un substrat de méthanisation à partir de biomasse fibreuse des produits fibreux, comprenant :
- une fosse (10) de dilution de la biomasse fibreuse pour former un mélange (M) contenant de la matière sèche (MS) fibreuse en suspension dans un liquide (L) et d'hydrolyse de la matière sèche dans le mélange ; et
- un dispositif de séparation de phase pour séparer de la matière sèche du liquide à partir du mélange.

11. installation selon la revendication 10, comprenant un dispositif de broyage amont (12) pour broyer la biomasse fibreuse avant sa dilution.

12. Installation selon la revendication 10 ou 11, comprenant un dispositif de broyage aval (24) pour broyer la matière sèche extraite du mélange.

13. Installation selon l'une quelconque des revendications 10 à 12, comprenant un dispositif de broyage en phase liquide (26) pour broyer la matière sèche dans le mélange.

14. Installation selon l'une quelconque des revendications 10 à 13 comprenant un dispositif d'ajustement de siccité pour mélanger la matière sèche (MS) à du liquide (L) séparé de la matière sèche par le dispositif de séparation et/ou du mélange (M) contenu dans la fosse.

15. Système de production de biogaz comprenant une installation de préparation (4) selon l'une quelconque des revendication 10 à 14 et un digesteur (6) anaérobie de méthanisation alimenté en substrat de méthanisation par l'installation de préparation.
